# EUROPÄISCHE PATENTSCHRIFT

(11) **EP 0 875 456 B1**
(45) Veröffentlichungstag und Bekanntmachung des Hinweises auf die Patenterteilung: **26.02.2003**
(21) Anmeldenummer: 98107213.5
(22) Anmeldetag: 21.04.1998
(51) Int. Cl.: B65B 29/10, B65B 55/02, A61F 2/46, A61M 5/315

(54) **Verfahren zur Herstellung von steril verpacktem Knochenzement**
Preparation method for sterile packaged bone cement
Procédé de préparation de ciment pour os emballé stérilement

(30) Priorität: 02.05.1997 DE 19718648
(43) Veröffentlichungstag der Anmeldung: 04.11.1998
(73) Patentinhaber: MERCK PATENT GmbH, 64293 Darmstadt (DE)
(72) Erfinder: Nies, Berthold, Dr., 64407 Fränkisch Crumbach (DE); Specht, Rainer, Dr., 64832 Babenhausen (DE)

(56) Entgegenhaltungen:
- EP-A- 0 313 851
- EP-A- 0 692 229
- WO-A-83/03587
- WO-A-94/16951
- GB-A- 2 272 197
- US-A- 4 463 875

## Beschreibung

Die vorliegende Erfindung betrifft ein Verfahren und eine Vorrichtung zur Herstellung von steril verpackten Knochenzementen. Insbesondere betrifft es ein Verfahren, bei welchem die Zementpulverkomponente in den zur Verpackung dienenden Behältern einer Gassterilisation unterworfen wird.

Die natürlichen Gelenke im menschlichen Körper unterliegen oft degenerierenden Veränderungen, die manchesmal auch aufgrund von Defekten nach krankheits- oder unfallbedingten operativen Eingriffen entstehen. Wenn diese Defekte zu weit fortgeschritten, irreversibel oder nicht mehr zu behandeln sind, wird es notwendig, die natürlichen Gelenke oder Knochen durch entsprechende Implantate zu ersetzen. Als Knochenersatzmaterialien sind beispielhaft Implantatformkörper verschiedenster Art oder Knochenverbindungselemente etwa in Form von Markraumnägeln, Knochenschrauben oder Osteosyntheseplatten zu nennen. Diese Knochenersatzmaterialien werden bei der Implantation mit Knochenzementen im natürlichen Knochen verankert.

Gängige Knochenzemente setzen sich aus einer Feststoffkomponente, die aus einem feinteiligen Polymerisat von Acryl- und/oder Methacrylsäureestern und weiteren Zusätzen wie Polymerisationskatalyatoren sowie gegebenenfalls Röntgenkontrastmitteln, Füllstoffen und Farbstoffen besteht, und aus einer flüssigen Komponente, die aus einem Acryl- und/oder Methacrylsäureestermonomeren und weiteren Zusätzen wie Polymerisationsbeschleunigern und Stabilisatoren besteht, zusammen. Die Polymerpulverkomponente des Zements besteht dabei vorzugsweise aus Granulatpartikeln mit Kugelform. Die Partikelgröße liegt vorzugsweise in einem engen Bereich oder ist weitgehend einheitlich.

Zum Gebrauch werden Feststoffkomponente und flüssige Komponente zu einer flüssigen bis halbfesten Paste vermengt, diese gegebenenfalls in eine gewünschte Form gebracht oder zur Einzementierung einer Prothese an dem Implantationsort appliziert. Die Aushärtung der Masse erfolgt durch die mit dem Vermischen der Komponenten induzierte Polymerisationsreaktion. Der Knochenzement wird zweckmäßigerweise in einer solchen Form bereitgestellt, dass getrennte Behältnisse mit aufeinander abgestimmten Mengen der beiden Komponenten als Packungseinheit zusammengefasst sind.

Es gibt verschiedene Möglichkeiten, die einzelnen Komponenten zur Knochenzementherstellung separat zur verpacken. Beispielweise kann das sterile Polymerpulver in Polyethylenbeutel abgepackt werden, die flüssige Komponente wird sterilfiltriert und beispielweise in Glasampullen abgefüllt.

Ferner gibt es Verpackungen, die aus flexiblen Folien bestehen und zwei oder mehr getrennte Kammern besitzen, welche durch entfernbare Verschlüsse voneinander getrennt sind. Zur Anmischung des fertigen Knochenzementes wird der Verschluss entfernt oder geöffnet und die Komponenten durch Kneten der flexiblen Beutel homogen vermischt. Ein solches System ist beispielsweise beschrieben in der Offenlegungsschrift WO 94/16951.

Bekannt sind auch solche Verpackungen, die wie die eben beschriebenen aufgebaut sind, aber zusätzlich ein mit dem Pulverbehälter verbundenes Vakuumreservoir besitzen und das Pulver unter Vakuum verpackt ist. Die Vakuumverpackung hat den Vorteil, dass beim Anmischen des Zementes weniger Luft in den Knochenzement eingearbeitet wird, was auf die Porosität des Zementes eine positiven Einfluss hat. Weiterhin wird durch das Vorhandensein eines Vakuumreservoirs die Durchmischung des Polymerpulvers mit dem Monomeren nach Öffnen der Verschlussvorrichtung wesentlich einfacher und effektiver, da durch den Unterdruck im Pulverbehältnis das Monomer regelrecht eingesaugt wird.

Die einzelnen Komponenten für die Herstellung des Knochenzementes müssen selbstverständlich steril abgepackt sein. Die üblichen Sterilisationsverfahren sind chemische Sterilisationen wie z.B. Ethylenoxid-Begasung und energiereiche Strahlung, meist γ-Bestrahlung oder β-Bestrahlung.

Bei den geschlossenen Zementiersystemen und insbesondere bei vakuumverpackten Zementpulvern werden bisher die einzelnen Komponenten entweder vor dem Abfüllen durch eine der genannten Sterilisationsverfahren sterilisiert und anschließend über Sterilfilter in die jeweiligen Behälter abgefüllt, d.h. es wird eine aseptische Abfüllung durchgeführt. Oder die Komponenten werden in den Packungen durch Bestrahlung sterilisiert.

Es ist jedoch bekannt, dass energiereiche Strahlung einen großen Einfluß auf die Eigenschaften des Knochenzementes hat. Insbesondere verändert sich die Viskosität in negativer Hinsicht und auch die Lagerstabilität nimmt nach eigenen Untersuchungen ab.

Aufgabe der vorliegenden Erfindung war es daher, eine Möglichkeit zu finden, steril verpackten Knochenzement herzustellen, wobei die Sterilität der Pulverkomponente nicht durch energiereiche Strahlung oder aseptische Abfüllung, bei welcher es doch zu einer mangelhaften Sterilität kommen kann, erzeugt wird.

Es wurden nun ein Verfahren und eine Vorrichtung gefunden, bei welchen ein Knochenzementpulver und die entsprechenden Behälter innen einer Gassterilisation unterworfen werden, und gegebenenfalls der Behälter enthaltend die Polymerpulverkomponente anschließend unter Beibehaltung der Sterilität evakuiert wird.

Gegenstand der Erfindung ist somit ein Verfahren zur Herstellung von steril verpacktem Knochenzement, das dadurch gekennzeichnet ist, dass das Polymerpulver in einen separaten, zur Verpackung dienenden Behälter, welcher über eine Verschlussvorrichtung mit einem weiteren Behälter für das Monomer verbunden ist, eingefüllt und über entsprechende Sterilfilter einer Gassterilisation unterworfen wird, wobei der zweite Behälter für das Monomer innen ebenfalls sterilisiert wird.

Gegenstand der Erfindung ist auch eine Vorrichtung zur Herstellung von steril verpacktem Knochenzement, wobei die Sterilität durch Gassterilisation erreicht wird, dadurch gekennzeichnet, dass diese Vorrichtung beinhaltet:
- ein zur Verpackung dienender Behälter für das Polymerpulver, der auf der einen Seite über eine Verschlussvorrichtung mit einem zweiten Behälter für das Monomer und auf der anderen Seite über eine Trennstelle mit einem Sterilfilter verbunden ist, und in welchem das Polymerpulver einer Gassterilisation unterzogen wird, wobei gegebenenfalls zwischen dem Polymerbehälter und der Trennstelle ein über einen Pulverfilter mit dem Polymerbehälter in Verbindung stehendes Ausdehnungs- oder Vakuumreservoir von ausreichender Größe angeordnet sein kann;
- ein zweiter Behälter für das Monomer, welcher auf einer Seite über die Verschlussvorrichtung mit dem Polymerbehälter und auf der anderen Seite über eine Trennstelle mit einem Sterilfilter verbunden ist, und welcher bei der Gassterilisation innen ebenfalls sterilisiert wird;
- gegebenenfalls ein Zuschlagstoffbehälter für eine weitere, flüssige oder feste Komponente, die dem Monomer vor der Vermischung mit dem Polymerpulver zugesetzt wird, welcher über eine Verschlussvorrichtung zwischen Monomerbehältnis und Sterilfilter angebracht ist, und welcher bei der Gassterilisation ebenfalls innen sterilisiert wird.

Die als Verpackung bzw. als Vakuumverpackung dienenden Behälter sind flexibel und bestehen vorzugsweise aus einem Folienmaterial, bei welchem es sich um ein Monomer-beständiges Polymer handelt. Es können für die vorliegende Erfindung ein- oder mehrschichtige Materialien, Komposite aus verschiedenen Polymeren und auch Komposite aus Polymeren mit nichtorganischen Lagen (z.B. Aluminiumfolie) eingesetzt werden.

Welches Material letztendlich verwendet wird, hängt davon ab, welche Stoffe abgepackt werden sollen, und unter welchen Bedingungen die Materialien angemischt werden. Für viele Substanzen ist eine Polyethylenfolie geeignet. Andere geeignete Materialien sind beispielsweise Teflon, Polyester, Nylon, Ethylenvinylalkohol, Metallfolie oder verschiedene Kombinationen der genannten Materialien. Üblicherweise werden thermoplastische Folien eingesetzt und die Behälter werden durch Verschweißen der Folienränder hergestellt.

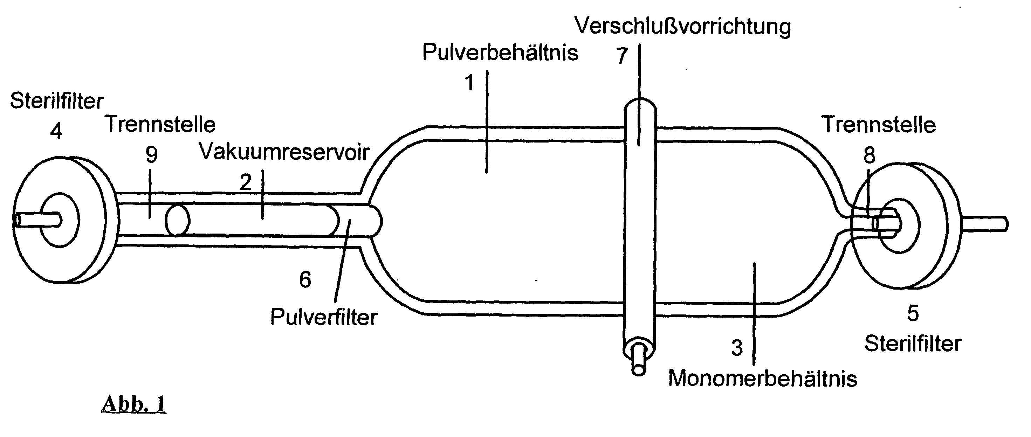

Anhand der Abbildung 1 soll in einfacher, schematischer Weise eine besonders bevorzugte Ausführungsform der zum erfindungsgemäßen Verfahren geeigneten Vorrichtung verdeutlicht werden. Der Pulverbehälter (1) steht gegebenenfalls auf der linken Seite über einen Pulverfilter (6) in Verbindung mit dem Ausdehnungs- oder Vakuumreservoir (2), an welchem sich ein Sterilfilter (4) befindet. Die Trennstelle (9) dient dazu, den Pulverbehälter nach der Durchführung des hier beschriebenen Sterilisationsverfahren gasdicht zu verschließen, z.B. durch Absiegeln. (1) kann aber auch direkt über (9) mit dem Sterilfilter verbunden sein.

Auf der rechten Seite ist der Pulverbehälter (1) über eine Verschlussvorrichtung (7) mit dem Monomerbehältnis (3) verbunden, welches auf der anderen Seite über eine Trennstelle (8) und einen Sterilfilter (5) verfügt.

Gegebenenfalls ist noch ein weiterer Behälter über eine Verschlussvorrichtung zwischen Monomerbehälter (3) und der Trennstelle (8) vorhanden, der eine dritte Komponente zur Herstellung des Knochenzementes oder zur Beimischung zum Knochenzement beinhalten kann.

Die Verschlussvorrichtungen zwischen den Behältern müssen leicht geschlossen und wieder geöffnet oder vollständig entfernt werden können. Es sind verschiedene solche Vorrichtungen bekannt. Vorzugsweise werden Klammern verschiedenster Art zur Abtrennung zweier Behälter eingesetzt, die bei Bedarf wieder leicht entfernt werden können. Ferner kann auch eine Hitze-Versiegelung oder ein Klebverschluss verwendet werden. Die Stärke dieser Verschlüsse muss dabei so gewählt werden, dass die Versiegelungen durch Druckanwendung auf einen der Behälter gebrochen werden können, ohne dass natürlich die Versiegelungsnähte der Behälter selber platzen.

Als besonders bevorzugte Verschlussvorrichtung dient beispielsweise eine etwa halbrunde Klammer mit einem Rundstab. Zwischen diese wird die Schlauchfolie eingeklemmt und bewirkt einen dichten Verschluss zwischen den jeweiligen Behältern. Zum Öffnen wird der Rundstab entfernt. Ähnliche Klammersysteme sind beispielsweise auch beschrieben in der WO 94/16951.

Das Ausdehnungs- oder Vakuumreservoir (2) ist über einen Pulverfilter (6) mit dem Polymerbehältnis verbunden, in einer anderen Ausführungsform kann das Ausdehnungs- oder Vakuumreservoir auch innerhalb des Polymerbehälters angeordnet sein.

Als Ausdehnungsreservoir wird dieses Reservoir bezeichnet, wenn die Verpackung unter Normaldruck durchgeführt wird, als Vakuumreservoir wird es bezeichnet, wenn eine Vakuumverpackung durchgeführt wird, d.h. wenn der Polymerbehälter nach der Durchführung der Gassterilisation und Entgasung zusätzlich evakuiert wird, was besonders bevorzugt ist.

Dieses Ausdehnungs- oder Vakuumreservoir muss jeweils eine ausreichende Größe besitzen, um die Gase aufzunehmen zu können, die beim Öffnen des Veschlusses zwischen Pulver- und Monomerbehälter und der Vermischung der beiden Komponenten noch auftreten können. Zur besseren Anmischung ist daher ein Vorhandensein eines solchen Ausdehnungs- oder Vakuumreservoir bevorzugt.

Insbesondere bevorzugt ist eine Verpackung unter Vakuum mit dem Vorhandensein eines Vakuumreservoirs. Durch den Druckunterschied zwischen dem evakuierten Pulverbehälter und dem Monomerbehälter erfolgt eine sehr gute Vermischung der beiden Komponenten. Ohne das Vakuumreservoir würde der zwischen den Pulverteilchen vorhandene Unterdruck oft nicht ausreichen, das gesamte Monomer in das Pulver einzusaugen. Die Funktion des Vakuumreservoirs liegt also darin, im Pulverbehältnis einen ausreichend niedrigen Druck aufrechtzuerhalten, bis die Anmischung vollständig erfolgt ist.

Nach der Anmischung wird das Vakuumreservoir oder auch im anderen Falle das Ausdehnungsreservoir dann abgetrennt, und der Zement kann zur Anwendung ausgetragen werden. Zweckmäßig ist es, einen solchen steril verpackten Zement mit einer Vorrichtung zum Ausbringen des Knochenzementes zu ergänzen. Entsprechende Vorrichtungen sind bekannt und gebräuchlich. Daher kann die den angemischten Zement enthaltende Packung auch in eine Knochenzementspritze bzw. -pistole eingesetzt werden, und der Zement auf diese Weise ausgetragen werden. Diese Methode ist aus der Literatur bekannt und beispielsweise in der WO 94/16951 beschrieben, dem Fachmann aber auch aus vielen anderen Quellen wohl bekannt.

Die hier verwendeten Steril- bzw. Pulverfilter sind Standardteile, die dem Stand der Technik entsprechen und jedem Fachmann auf diesem Gebiet bekannt sind. Eine weitere Erläuterung ist daher nicht notwendig.

In einer bevorzugten Ausführungsform wird das erfindungsgemäße Sterilisations-verfahren nun folgerndermassen durchgeführt. Zunächst wird der Sterilfilter (5) angebracht, dann wird die Verschlussvorrichtung (7) verschlossen. Anschließend erfolgt das Befüllen des Pulverbehältnisses (1) von der rechten Seite ehe der Pulverfilter (6) und der Sterilfilter (4) nacheinander eingebracht werden. Das pulvergefüllte Behältnis wird schließlich der Sterilisation unterworfen. Dazu wird das zu sterilisierende Gut in eine Kammer gebracht und das Sterilisationsgas, vorzugsweise Ethylenoxid-Gas, in die Kammer eingelassen. Dieses Gas wirkt über mehrere Stunden auf das zu sterilisierende Gut ein; die Dauer der Sterilisation kann zwischen einer Stunde und 24 Stunden liegen. Das Gas kann dabei über die Sterilfilter in alle Hohlräume der Behältnisse eindringen. Das bedeutet, dass auch das Monomerbehältnis (3) innen sterilisiert wird.

Eine andere, besonders bevorzugte Variante besteht darin, dass man wie eben beschrieben vorgeht und das zu sterilisierende Gut in eine Kammer gebracht wird. Dann wird die Kammer jedoch evakuiert, bevor das Sterilisationsgas in die Kammer eingelassen wird. Dann geht man weiter vor, wie oben beschrieben.

Danach wird vorzugsweise das Ethylenoxid-Gas abgepumpt bzw. ausgelassen. Dazu wird das sterilisierte Gut in eine Entgasungskammer gebracht und dort über einen Zeitraum von 1 - 50 Tagen entgast, teils unter Normalbedingungen, teils mit Vakuum. Sobald die Restgaskonzentration von ca. 1 ppm erreicht ist, wird der Pulverbehälter auf der Linken Seite auch an der Trennstelle (9) gasdicht verschlossen, z.B. durch Absiegeln, also durch ein thermisches Verschmelzen des Folienbeutels (z.B. mit einem Folienschweißgerät).

Im Hinblick auf die Zementqualität und den Anmischungsvorgang wird jedoch vor dem Absiegeln an der Trennstelle (9) die noch vorhandene Luft herausgedrückt oder der Pulverbehälter wird über den linken Sterilfilter evakuiert. Nach Erreichen des gewünschten Vakuums, das vorzugsweise bei 0,01 - 0,2 bar liegt, wird der Behälter dann wie eben beschrieben an der Trennstelle (9) verschlossen. Diese Ausführungsvariante der Evakuierung, die zu einer Vakuumverpackung der Polymerkomponente führt, ist ganz besonders bevorzugt.

Anschließend erfolgt nun das Abfüllen des sterilen Monomers durch den Sterilfilter (5), z.B. über eine Durchstechkanüle, in das innen sterilisierte Monomerbehältnis. Vorzugsweise wird dann die verbleibende Luft entfernt, was durch Herausdrücken oder Absaugen passieren kann, dann wird auch dieser Behälter an der Trennstelle (8) verschlossen, z.B. durch Absiegeln.

Damit sind die Zementkomponenten sterilisiert und verpackt bzw. vakuumverpackt. In einer bevorzugten Ausführungsform der Erfindung wird anschließend das Behältnis in der Sekundärpackung auch außen durch Ethylenoxid-Begasung sterilisiert.

Eine weitere Ausführungsform der vorliegenden Erfindung, die ebenfalls Gegenstand der Erfindung ist, weist einen dritten Behälter (Zuschlagstoffbehälter) auf, der sich dann vorzugsweise zwischen Sterilfilter (5) bzw. Trennstelle (8) und dem Monomerbehälter (3) befindet, wobei die beiden Behälter durch einen entfernbaren Verschlussmechanismus, vergleichbar mit der Verschlussvorrichtung (7), miteinander verbunden sind. Dieser Zuschlagstoffbehälter kann dann eingesetzt werden, falls eine weitere Komponenten zur Anmischung des Zementes zugesetzt werden soll. Diese dritte Komponente kann flüssiger oder fester Natur sein und wird dem Monomer vor der Vermischung mit dem Pulver zugesetzt.

Das Sterilisationsverfahren wird wie schon beschrieben durchgeführt. Nur wird nach Abfüllen des Monomers und gegebenenfalls nach Entfernen der Luft aus diesem Behälter der Verschluss zwischen Monomer- und Zuschlagstoffbehälter verschlossen, anschließend die sterilisierte dritte Komponente über den Sterilfilter eingefüllt und an der Trennstelle (8) abgesiegelt, gegebenenfalls auch hier nach Entfernen der Luft.

Zur Anmischung wird dann zunächst die Verbindung zwischen dem Zuschlagstoff- und dem Monomerbehälter geöffnet. Nach homogener Vermischung wird dann die Verbindung zwischen Pulver und Monomer geöffnet und eine homogene Zementmischung herbeigeführt.

Je nach Zementrezeptur kann es bei der Anmischung auch nützlich oder erforderlich sein, die Mischung durch Kneten der flexiblen Behälter zu verbessern

Die Menge der einzelnen Komponenten sind zweckmäßigerweise genau aufeinander abgestimmt. Bei den üblichen Zementen liegt das Verhältnis zwischen Pulver und Monomer in einem Bereich zwischen 1,5:1 und 2,5:1.

Das Knochenzementpulver enthält dabei üblicherweise die bekannten Polymere sowie Polymerisationsstarter (die im Polymer enthalten sein können), ferner können auch Röntgenkontrastmittel, Füllstoffe, Farbstoffe und gegebenenfalls auch Antibiotika oder weitere Wirkstoffe enthalten sein. Die Monomerkomponente kann noch Polymerisationsbeschleuniger oder auch Stabilisatoren enthalten.

Bei der Herstellung bestimmter Knochenzemente kann es wünschenswert oder auch notwendig sein, dritte Komponenten, wie z.B. Wirkstoffe oder die schon erwähnten anderen Zusatzstoffe, separat in den beschriebenen Zuschlagstoffbehälter abzufüllen. In diesem Falle wird dann wie schon gesagt, diese dritte Komponente zunächst mit dem Monomer vermischt, bevor die Anmischung mit dem Polymerpulver erfolgt. Auch diese dritte Komponente wird zweckmäßigerweise in der abgestimmten Menge abgepackt. Beispielsweise kann eine dritte Komponente in einer Menge zugesetzt werden, die im Bereich zwischen 5 % und 20 % des Monomergewichtes liegt.

Als Beispiel kann hier die Herstellung eines MTX-Zementes angeführt werden. MTX-Zement ist ein Zement, der neben den üblichen Zementkomponenten als weitere Komponente das Zytostatikum Methotrexat enthält. Diese Komponente kann als Lösung, beispielsweise gelöst in Vinylpyrrolidon, zugegeben werden. Dieses Zytostatikum wird nach der Implantation des Zementes an der Zementmatrix freigesetzt und kann zur lokalen Behandlung z.B. von Knochentumoren eingesetzt werden.

Die vorliegende Erfindung liefert somit ein hervorragendes Verfahren zur sterilen Verpackung oder sterilen Vakuumverpackung der Zementkomponenten, wobei die Pulverkomponente in dem zur Verpackung dienenden Behälter durch Gassterilisation sterilisiert wird. Die negativen Effekte, die durch eine Sterilisation mit energiereicher Strahlung auftreten, sind hier überwunden. Dadurch weisen die angemischten Zemente eine wesentlich bessere Qualität auf.

Auch ohne weitere Ausführung wird davon ausgegangen, dass ein Fachmann die obige Beschreibung in weitesten Umfang nutzen kann. Die bevorzugten Ausführungsformen sind deswegen lediglich als beschreibende, keineswegs als in irgendeine Weise limitierte Offenbarung aufzufassen.

Die vollständige Offenbarung aller vor- und nachstehend aufgeführten Anmeldungen, Patente und Veröffentlichungen sind durch Bezugnahme in diese Anmeldung eingeführt.

### Beispiel 1

Es wird eine Vorrichtung analog zu Abb. 1 benutzt.

Man füllt 40 g des Pulvers, bestehend aus Polymethacrylat (PMA) / Polymethyl-methacrylat (PMMA) - Coploymer, ZrO₂, Benzoylperoxid (BPO) in die die oben bevorzugt beschriebene Apparatur. Diese Apparatur wird in eine Kammer gebracht, die Kammer wird evakuiert und das Ethylenoxid-Gas wird in die Kammer eingelassen. Man lässt das Gas etwa 20 Stunden auf das zu sterilisierende Gut einwirken.

Danach wird das Ethylenoxid-Gas abgepumpt. Die sterilisierte Vorrichtung wird in eine Entgasungskammer gebracht und dort unter Normalbedingungen entgast, bis die Restgaskonzentration ca. 1 ppm beträgt. Anschließend evakuiert man über den Sterilfilter (4) den Pulverbehälter. Nach Erreichen des gewünschten Vakuums wird an der Trennstelle (9) der Pulverbehälter abgesiegelt.

Danach werden 20 ml der sterilen Monomerkomponente MMA + Dimethylpara-toluidin (DMPT) durch den Sterilfilter (5) in das innen ebenfalls sterilisierte Monomerbehältnis eingefüllt. Nach Herausdrücken der verbleibenden Luft wird auch dieses Behältnis durch Absiegeln an der vorgesehenen Trennstelle (8) verschlossen.

Damit sind die Zementkomponenten steril vakuumverpackt. Das Behältnis kann anschließend in der Sekundärpackung auch noch außen durch eine Ethylenoxid-Begasung sterilisiert werden.

### Beispiel 2

Man benutzt eine Apparatur analog zu Abb. 1, ergänzt durch einen Zuschlagstoffbehälter, der über eine Verschlussvorrichtung analog zu (7) auf der linken Seite des Monomerbehälters angebracht ist.

Wie in Beispiel 1 beschrieben, füllt man zunächst 40 g der Pulverkomponente, enthaltend PMA / PMMA - Copolymer, ZrO₂, BPO, ab und verfährt analog zu Beispiel 1 bis zur Evakuierung und Absiegelung des Pulverbehälters. Dann werden 20 ml der Monomerkomponente, enthaltend MMA + DMPT, wie in Beispiel 1 abgefüllt, dann wird nach Herausdrücken der verbleibenden Luft die Verschlussvorrichtung zwischen Monomerbehälter und dem Zuschlagstoffbehälter geschlossen. Anschließend wird die sterilisierte dritte Komponente, 2 ml Methotrexat-Lösung über den Sterilfilter (5) eingefüllt und danach die Trennstelle (8) abgesiegelt.

Falls gewünscht, kann das Behältnis in der Sekundärpackung außen durch eine Ethylenoxid-Begasung sterilisiert werden.

## Patentansprüche

1. Verfahren zur Herstellung von steril verpacktem Knochenzement, **dadurch gekennzeichnet, dass** das Polymerpulver in einen separaten, zur Verpackung dienenden Behälter (1), welcher über eine Verschlussvorrichtung (7) mit einem weiteren Behälter (3) für das Monomer verbunden ist, eingefüllt und über entsprechende Sterilfilter (4,5) einer Gassterilisation unterworfen wird, wobei der zweite Behälter für das Monomer innen ebenfalls sterilisiert wird.

2. Verfahren nach Anspruch 1, **dadurch gekennzeichnet, dass** anschließend an die Gassterilisation die Behälter (1,3) über die Sterilfilter (4,5) entgast werden und dann der Pulverbehälter (1) an der vorgesehenen Trennstelle (8) zwischen Pulverbehälter (1) und Sterilfilter (4) verschlossen wird.

3. Verfahren nach Anspruch 1 oder 2, **dadurch gekennzeichnet, dass** anschließend an die Gassterilisation und/oder die Entgasung der Pulverbehälter (1) über den mit dem Pulverbehâlter verdundenen Sterilfilter (4) evakuiert wird, und anschließend der Pulverbehälter (1) an der vorgesehenen Trennstelle (9) zwischen Behälter (1) und Sterilfilter (4) verschlossen wird.

4. Verfahren nach einem der Ansprüche 1 bis 3, **dadurch gekennzeichnet, dass** in einem weiteren Schritt das sterile Monomer über den mit dem Monomerbehälter verbundenen Sterilfilter (5) in das innen sterile Monomerbehältnis (3) eingefüllt wird, und dieses ebenfalls an der vorgesehenen Trennstelle (8) zwischen Sterilfilter (5) und Monomerbehältnis (3) verschlossen wird.

5. Verfahren nach einem der Ansprüche 1 bis 4, **dadurch gekennzeichnet, dass** noch eine dritte, flüssige oder feste Komponente verwendet werden kann, wobei sich diese weitere Komponente in einem dritten Behälter befindet, der sich in diesem Falle dann zwischen Sterilfilter (5) und Monomerbehälter (3) befindet, verbunden durch eine Verschlussvorrichtung (7).

6. Verfahren nach einem der Ansprüche 1 bis 5, **dadurch gekennzeichnet, dass** die Gassterilisation mittels Ethylenoxid-Gas durchgeführt wird.

7. Verfahren nach einem der Ansprüche 1 bis 6, **dadurch gekennzeichnet, dass** ein in Verbindung mit dem Pulverbehälter (1) stehendes Ausdehnungs- oder Vakuumreservoir (2) von ausreichender Größe vorhanden ist.

8. Verfahren nach einem der Ansprüche 1 bis 7, **dadurch gekennzeichnet, dass** zum Schluss die steril verpackten Zementkomponenten in der Sekundärpackung auch außen sterilisiert werden.

9. Vorrichtung zur Herstellung von steril verpacktem Knochenzement, wobei die Sterilität durch Gassterilisation erreicht wird, **dadurch gekennzeichnet, dass** diese Vorrichtung beinhaltet:
- ein zur Verpackung dienender Behälter (1) für das Polymerpulver, der auf der einen Seite über eine Verschlussvorrichtung (7) mit einem zweiten Behälter (3) für das Monomer und auf der anderen Seite über eine erste Trennstelle (9) mit einem ersten Sterilfilter (4) verbunden ist, und in welchem das Polymerpulver über den ersten Sterilfilter (4) einer Gassterilisation unterzogen wird;
- ein zweiter Behälter für das Monomer, welcher auf einer Seite über die Verschlussvorrichtung mit dem Polymerbehälter und auf der anderen Seite über eine zweite Trennstelle mit einem zweiten Sterilfilter verbunden ist, und welcher bei der Gassterilisation innen ebenfalls sterilisiert wird.

10. Vorrichtung nach Anspruch 9, **dadurch gekennzeichnet, dass** zwischen dem Polymerbehälter (1) und der ersten Trennstelle (9) ein über einen Pulverfilter mit dem Polymerbehälter in Verbindung stehendes Ausdehnungs- oder Vakuumreservoir (2) von ausreichender Größe angeordnet ist.

11. Vorrichtung nach einem der Ansprüche 9 und/oder 10, **dadurch gekennzeichnet, dass** sie zusätzlich einen Zuschlagstoffbehälter für eine weitere, flüssige oder feste Komponente, die dem Monomer von der Vermischung mit dem Polymerpulver zugesetzt wird, welcher über eine Verschlussvorrichtung (7) mit dem Monomerbehältnis (3) verbunden ist, und welcher bei der Gassterilisation ebenfalls innen sterilisiert wird, beinhaltet.

12. Vorrichtung nach einem der Ansprüche 9 bis 11, **dadurch gekennzeichnet, dass** die zur Verpackung dienenden Behälter auch zur Vakuumverpackung geeignet sind und aus flexiblen Polymerfolien bestehen.

## Claims

1. Process for the production of sterile-packed bone cement, **characterised in that** the polymer powder is introduced into a separate container (1) serving for packaging, which is connected via a closure device (7) to a further container (3) for the monomer, and is subjected to gas sterilisation via corresponding sterile filters (4, 5), where the second container for the monomer is likewise sterilised on the inside.

2. Process according to Claim 1, **characterised in that** after the gas sterilisation, the containers (1, 3) are degassed via the sterile filters (4, 5), and the powder container (1) is then sealed at the intended separating point (8) between the powder container (1) and the sterile filter (4).

3. Process according to Claim 1 or 2, **characterised in that** after the gas sterilisation and/or the degassing, the powder container (1) is evacuated via the sterile filter (4) connected to the powder container, and subsequently the powder container (1) is sealed at the intended separating point (9) between the container (1) and the sterile filter (4).

4. Process according to one of Claims 1 to 3, **characterised in that** in a further step, the sterile monomer is introduced into the monomer container (3), which is sterile on the inside, via the sterile filter (5), which is connected to the monomer container, and the monomer container is likewise sealed at the intended separating point (8) between the sterile filter (5) and the monomer container (3).

5. Process according to one of Claims 1 to 4, **characterised in that** a third, liquid or solid component can also be used, where this further component is located in a third container which is in this case located between the sterile filter (5) and the monomer container (3), connected by a closure device (7).

6. Process according to one of Claims 1 to 5, **characterised in that** the gas sterilisation is carried out by means of ethylene oxide gas.

7. Process according to one of Claims 1 to 6, **characterised in that** an expansion or vacuum reservoir (2) of sufficient size which is connected to the powder container (1) is present.

8. Process according to one of Claims 1 to 7, **characterised in that** the sterile-packed cement components in the secondary pack are finally also sterilised on the outside.

9. Apparatus for the production of sterile-packed bone cement in which the sterility is achieved by gas sterilisation, **characterised in that** this apparatus comprises:
- a polymer powder container (1), serving for packaging, which is connected on one side via a closure device (7) to a second container (3) for the monomer and on the other side via a first separating point (9) to a first sterile filter (4), and in which the polymer powder is subjected to gas sterilisation via the first sterile filter (4);
- a second container for the monomer, which is connected on one side via the closure device to the polymer container and on the other side via a second separating point to a second sterile filter, and which is likewise sterilised on the inside during the gas sterilisation.

10. Apparatus according to Claim 9, **characterised in that** an expansion or vacuum reservoir (2) of sufficient size, which is connected to the polymer container via a powder filter, is arranged between the polymer container (1) and the first separating point (9).

11. Apparatus according to one of Claims 9 and/or 10, **characterised in that** it additionally comprises an additive container for a further, liquid or solid component which is added to the monomer before mixing with the polymer powder, which container is connected to the monomer container (3) via a closure device (7) and which is likewise sterilised on the inside during the gas sterilisation.

12. Apparatus according to one of Claims 9 to 11, **characterised in that** the containers serving for packaging are also suitable for vacuum packaging and consist of flexible polymer films.

## Revendications

1. Procédé pour la production de ciment à os conditionné de manière stérile, **caractérisé en ce que** la poudre polymère est placée dans un réservoir (1) distinct, utilisé pour le conditionnement, lequel réservoir est relié avec un autre réservoir (3) destiné au monomère à l'aide d'un dispositif de fermeture (7), et ladite poudre est soumise à une stérilisation au gaz par le biais de filtres stériles correspondants (4, 5), le second réservoir destiné au monomère étant également stérilisé de manière interne.

2. Procédé selon la revendication 1, **caractérisé en ce que** les réservoirs (1, 3) sont dégazés consécutivement à la stérilisation au gaz par le biais des filtres stériles (4, 5) puis le réservoir de poudre (1) est fermé au niveau du point de coupure prévu (8) entre le réservoir de poudre (1) et le filtre stérile (4).

3. Procédé selon la revendication 1 ou 2, **caractérisé en ce que** le réservoir de poudre (1) est purgé au moyen du filtre stérile (4) relié avec le réservoir de poudre consécutivement à la stérilisation au gaz et / au dégazage, puis le réservoir de poudre (1) est fermé au niveau du point de coupure prévu (9) entre le réservoir de poudre (1) et le filtre stérile (4).

4. Procédé selon l'une des revendications 1 à 3, **caractérisé en ce que** dans une autre étape le monomère stérile est versé dans le réservoir du monomère (3) stérile de manière interne au moyen du filtre stérile (5) relié avec le réservoir de monomère et ce réservoir est également fermé au niveau du point de coupure prévu (8) entre le filtre stérile (5) et le réservoir de monomère (3).

5. Procédé selon l'une des revendications 1 à 4, **caractérisé en ce qu'**un troisième composant fluide ou solide peut être utilisé, ce troisième autre composant se trouvant dans un troisième réservoir, qui se trouve dans ce cas entre le filtre stérile (5) et le réservoir de monomère (3), relié au moyen d'un dispositif de fermeture (7).

6. Procédé selon l'une des revendications 1 à 5, **caractérisé en ce que** la stérilisation au gaz est réalisée au moyen d'oxyde d'éthylène gazeux.

7. Procédé selon l'une des revendications 1 à 6, **caractérisé en ce qu'**un réservoir sous vide ou d'expansion (2) de dimensions suffisantes, qui est relié avec le réservoir de poudre (1), existe.

8. Procédé selon l'une des revendications 1 à 7, **caractérisé en ce qu'**à la fin les composants du ciment conditionnés de manière stérile sont également stérilisés à 1'extérieur dans 1'emballage secondaire.

9. Dispositif pour la fabrication de ciment à os conditionné de façon stérile, la stérilité étant atteinte par la stérilisation au gaz, **caractérisé en ce que** ce dispositif comprend
- un réservoir (1) pour la poudre polymère utilisé pour le conditionnement, lequel réservoir est d'une part relié à un second réservoir (3) pour le monomère au moyen d'un dispositif de fermeture (7) et d'autre part à un premier filtre stérile (4) par le biais d'un premier point de coupure (9) et dans lequel la poudre polymère est soumise à une stérilisation au gaz au moyen d'un premier filtre stérile (4);
- un second réservoir pour le monomère, lequel réservoir est d'une part relié au réservoir de polymère au moyen du dispositif de fermeture et d'autre part à un second filtre stérile par le biais d'un second point de coupure et lequel réservoir est également stérilisé de manière interne lors de la stérilisation au gaz.

10. Dispositif selon la revendication 9 **caractérisé en ce qu'**il existe, entre le réservoir de polymère (1) et le premier point de coupure (9), un réservoir sous vide ou d'expansion (2) de dimensions suffisantes qui est relié au réservoir de polymère à l'aide d'un filtre à poudre.

11. Dispositif selon l'une des revendications 9 et / ou 10, **caractérisé en ce qu'**il comporte en plus un réservoir d'adjuvant pour un autre composant fluide ou solide, qui est ajouté au monomère entrant dans le mélange avec la poudre polymère, lequel réservoir est relié au réservoir de monomère (3) à l'aide d'un dispositif de fermeture (7) et lequel réservoir est également stérilisé de façon interne lors de la stérilisation au gaz.

12. Dispositif selon l'une des revendications 9 à 11, **caractérisé en ce que** les réservoirs utilisés pour le conditionnement sont également appropriés à un conditionnement sous vide et qu'ils se composent de feuilles en polymère flexibles.
